# EUROPEAN PATENT SPECIFICATION

(11) **EP 1 527 094 B1**
(45) Date of publication and mention of the grant of the patent: **16.07.2008**
(21) Application number: 03792268.9
(22) Date of filing: 07.08.2003
(51) Int. Cl.: C07K 14/505, C12N 15/12, A61K 38/18, G01N 33/68

(54) **T-CELL EPITOPES IN ERYTHROPOIETIN**
T-ZELLEN-EPITOPE IN ERYTHROPOIETIN
EPITOPES DE LYMPHOCYTES T DANS L'ERYTHROPOIETINE

(30) Priority: 09.08.2002 EP 02017914
(43) Date of publication of application: 04.05.2005
(73) Proprietor: Merck Patent GmbH, 64293 Darmstadt (DE)
(72) Inventor: Baker, Matthew, Cambridgeshire GB6 1HR (GB); Carr, Francis J., Balmedie, Aberdeenshire AB23 8XU (GB)
(86) International application number: PCT/EP2003/008725
(87) International publication number: WO 2004/018515

(56) References cited:
- EP-A- 0 267 678
- EP-A- 0 357 804
- EP-A- 0 410 246
- EP-A- 0 539 167
- EP-A- 1 013 288
- WO-A-00/34317
- WO-A-02/20034
- WO-A-02/062843
- WO-A-02/069232

## Description

### FIELD OF THE INVENTION

The present invention relates to the field of immunology. The invention identifies determinants on human erythropoietin (EPO) able to evoke an immune response. In particular the invention is concerned with the identification of epitopes for T-cells in human EPO. The invention relates furthermore to T-cell epitope peptides derived EPO by means of which it is possible to create modified EPO variants with reduced immunogenicity.

### BACKGROUND OF THE INVENTION

There are many instances whereby the efficacy of a therapeutic protein is limited by an unwanted immune reaction to the therapeutic protein. Several mouse monoclonal antibodies have shown promise as therapies in a number of human disease settings but in certain cases have failed due to the induction of significant degrees of a human anti-murine antibody (HAMA) response [Schroff, R. W. et al (1985) Cancer Res. 45: 879-885; Shawler, D.L. et al (1985) J. Immunol. 135: 1530-1535]. For monoclonal antibodies, a number of techniques have been developed in attempt to reduce the HAMA response [WO 89/09622; EP 0239400; EP 0438310; WO 91/06667]. These recombinant DNA approaches have generally reduced the mouse genetic information in the final antibody construct whilst increasing the human genetic information in the final construct. Notwithstanding, the resultant "humanised" antibodies have, in several cases, still elicited an immune response in patients [Issacs J.D. (1990) Sem. Immunol. 2: 449, 456; Rebello, P.R. et al (1999) Transplantation 68: 1417-1420].

Antibodies are not the only class of polypeptide molecule administered as a therapeutic agent against which an immune response may be mounted. Even proteins of human origin and with the same amino acid sequences as occur within humans can still induce an immune response in humans. Notable examples amongst others include the therapeutic use of granulocyte-macrophage colony stimulating factor [Wadhwa, M. et al (1999) Clin. Cancer Res. 5: 1353-1361] and interferon alpha 2 [Russo, D. et al (1996) Bri. J. Haem. 94: 300-305; Stein, R. et al (1988) New Engl. J. Med. 318: 1409-1413]. In such situations where these human proteins are immunogenic, there is a presumed breakage of immunological tolerance that would otherwise have been operating in these subjects to these proteins.

A prominent recent example of this problem is seen with the therapeutic use of recombinant human erythropoietin (EPO). This is a critical glycoprotein growth factor as it promotes red blood cell formation *in vivo.* The protein is used therapeutically in the treatment of anaemic patients on dialysis and other patients in whom anaemia is a problem. The protein has proven safe and effective in the management of anaemia for the large majority of patients. However in 1997 Prabhakar and Muhlfelder [Prabhakar, S. & Muhlfelder, T. Clin. Nephrol. 47: 331-335] described a single case of pure red cell aplasia in the face of high titres of anti-EPO antibodies. Subsequently multiple other cases of pure red cell aplasia linked to the development of antibodies to the recombinant EPO have been reported. It has been concluded that the induced antibodies cross-react with the endogenous protein leading to a complete blockade of the differentiation of red blood cells (see Indiveri F. & Murdaca, G for review; Rev. Clin. Exp. Hematol. (2002) Suppl. 1: 7-11). The patients survive only by frequent blood transfusions and although antibody levels decrease when the treatment is stopped around half of effected patients remain transfusion dependent.

A sustained antibody response to a therapeutic protein such as EPO requires the stimulation of T-helper cell proliferation and activation. T-cell stimulation requires the establishment of a T-cell synapse between a T-cell and an antigen presenting cell (APC). At the core of the synapse is the T-cell receptor (TCR) on the T-cell engaged with a peptide MHC class II complex on the surface of the APC. The peptide is derived from the intracellular processing of the antigenic protein. Peptide sequences from protein antigens that can stimulate the activity of T-cells via presentation on MHC class II molecules are the termed "T-cell epitopes". Such T-cell epitopes are commonly defined as any amino acid residue sequence with the ability to bind to MHC Class II molecules. Implicitly, a "T-cell epitope" means an epitope which when bound to MHC molecules can be recognised by a TCR, and which can, at least in principle, cause the activation of these T-cells by engaging a TCR to promote a T-cell response. It is understood that for many proteins a small number of T-helper cell epitopes can drive T-helper signalling to result in sustained, high affinity, class-switched antibody responses to what may be a very large repertoire of exposed surface determinants on the therapeutic protein.

T-cell epitope identification is recognised as the first step to epitope elimination, and it is highly desired to identify T-cell epitopes in therapeutic proteins such as EPO. Patent applications WO98/52976 and WO00/34317 teach computational threading approaches to identifying polypeptide sequences with the potential to bind a sub-set of human MHC class II DR allotypes. In these teachings, predicted T-cell epitopes are removed by the use of judicious amino acid substitution within the protein of interest. However with this scheme and other computationally based procedures for epitope identification [Godkin, A.J. et al (1998) J. Immunol. 161: 850-858; Sturniolo, T. et al (1999) Nat. Biotechnol. 17: 555-561], peptides predicted to be able to bind MHC class II molecules may not function as T-cell epitopes in all situations, particularly, *in vivo* due to the processing pathways or other phenomena. In addition, the computational approaches to T-cell epitope prediction have in general not been capable of predicting epitopes with DP or DQ restriction.

Equally, *in vitro* methods for measuring the ability of synthetic peptides to bind MHC class II molecules, for example using B-cell lines of defined MHC allotype as a source of MHC class II binding surface [Marshall K.W. et al. (1994) J. Immunol. 152:4946-4956; O'Sullivan et al (1990) J. Immunol. 145: 1799-1808; Robadey C. et al (1997) J. Immunol 159: 3238-3246], may be applied to MHC class II ligand identification. However, such techniques are not adapted for the screening multiple potential epitopes to a wide diversity of MHC allotypes, nor can they confirm the ability of a binding peptide to function as a T-cell epitope.

Recently techniques exploiting soluble complexes of recombinant MHC molecules in combination with synthetic peptides have come into use [Kem, F. et al (1998) Nature Medicine 4:975-978; Kwok, W.W. et al (2001) TRENDS in Immunol. 22:583-588]. These reagents and procedures are used to identify the presence of T-cell clones from peripheral blood samples from human or experimental animal subjects that are able to bind particular MHC-peptide complexes and are not adapted for the screening multiple potential epitopes to a wide diversity of MHC allotypes.

Biological assays of T-cell activation remain the best practical option to providing a reading of the ability of a test peptide/protein sequence to evoke an immune response. Examples of this kind of approach include the work of Petra et al using T-cell proliferation assays to the bacterial protein staphylokinase, followed by epitope mapping using synthetic peptides to stimulate T-cell lines [Petra, A.M. et al (2002) J. Immunol. 168: 155-161]. Similarly, T-cell proliferation assays using synthetic peptides of the tetanus toxin protein have resulted in definition of immunodominant epitope regions of the toxin [Reece J.C. et al (1993) J. Immunol. 151: 6175-6184]. WO99/53038 discloses an approach whereby T-cell epitopes in a test protein may be determined using isolated sub-sets of human immune cells, promoting their differentiation *in vitro* and culture of the cells in the presence of synthetic peptides of interest and measurement of any induced proliferation in the cultured T-cells. The same technique is also described by Stickler et al [Stickler, M.M. et al (2000) J. Immunotherapy 23:654-660], where in both instances the method is applied to the detection of T-cell epitopes within bacterial subtilisin. Such a technique requires careful application of cell isolation techniques and cell culture with multiple cytokine supplements to obtain the desired immune cell sub-sets (dendritic cells, CD4+ and or CD8+ T-cells).

In a variation of these approaches, Hiemstra et al [Hiemstra, H.S. (1997) Proc. Natl. Acad. Sci USA 94: 10313-10318] have described a procedure for identifying a peptide epitope capable of stimulating a known T-cell, such a process is valuable in the detection of autoreactive T-cell clones for which the (auto)antigen is unknown.

The above examples and other biological assays involving technical variations on the theme of measuring an *in vitro* T-cell activation event, usually by the measurement of an induced proliferation response, abound. However, none of the procedures provide a unified scheme for the detection of biologically relevant epitopes in proteins of human origin nor are readily applicable to the detection of epitopes of significance to a wide population of MHC allotypes. The present invention provides a scheme for the identification and of T-cell epitopes in human EPO and EPO analogues in which the T-cell epitopes are compromised in their ability to interact with human MHC class II molecules.

Potential MHC class II ligands within the human EPO sequence have been described previously by the present inventors within WO 02/062843. In contrast to the present invention, the dataset of possible MHC class II ligands disclosed within WO 02/062843 is derived using computational means only, is very large and represents the universe of possible MHC ligands. For reasons such as the requirement for proteolytic processing of the complete EPO protein and other physiologic steps leading to the presentation of EPO peptides *in vivo,* it is clear that only a minor sub-set of the entire repertoire of peptides will have ultimate biological relevance and the present invention in the form of the EPO T-cell epitope map, is conceived to address this short fall in the art.

As stated previously, recombinant EPO is used as an effective treatment of anaemia resulting from chronic renal failure. EPO is a glycoprotein hormone involved in the maturation of erythroid progenitor cells into erythrocytes. Naturally occurring EPO is produced by the liver during foetal life and by the kidney of adults. The hormone circulates in the blood to stimulate production of red blood cells in bone marrow. Anaemia is almost invariably a consequence of renal failure due to decreased production of EPO from the kidney. The production of EPO using recombinant DNA techniques has been described previously [Jacobs et al Nature, 313: 806-810; Lin, F.-K. et al (1985) Proc. Natl. Acad. Sci. U.S.A. 82:7580-7585] and therapeutic quantities may be produced for example according to the process described in Kirin-Amgen PCT Publication WO 85/02610 and other examples.

The mature amino acid sequence of human EPO contains 166 amino acid residues and depicted in single-letter code comprises the following sequence:

APPRLICDSRVLERYLLEAKEAENITTGCAEHCSLNENITVPDTKVNFYAWKRMEVGQQAVEVWQ
GLALLSEAVLRGQALLVNSSQPWEPLQLHVDKAVSGLRSLTTLLRALGAQKEAISPPDAASAAPL
RTITADTFRKLFRVYSNFLRGKLKLYTGEACRTGDR

Recombinant human EPO (expressed in mammalian cells) contains three N-linked and one O-linked oligosaccharide chains each containing terminal sialic acid residues. The latter are significant in enabling EPO to evade rapid clearance from the circulation by the hepatic asialoglycoprotein binding protein. Several studies have been conducted in which mutational modification of the protein has been applied to gain understanding of the structure and function of the molecule. For example studies by Yamaguchi [Yamaguchi, K. et al. (1991) J. Biol. Chem. 266: 20434-20439], Delorme [Delorme, E. et al.(1992) Biochemistry 31: 9871-9876] and by Bill [Bill, R. et al (1995) Biochim. Biophys. Acta. 1261: 35-43] have focussed on the sites of N-linked and O-linked glycosylation within the protein by making substitutions at N24, N38, N83 and S126. This work has indicated that some N-linked glycosylation is required for EPO activity, the N-linked sugars in particular increase the apparent molecular weight of EPO and prolong its circulating half-life. In the case of Bill et al, substitution was to cysteine for N24, N38 and N83 resulting in greatly reduced functional activity.

Cysteine substitution is also disclosed by WO99/03887 where the purpose is to provide cysteine-added EPO variants and their use in preparing conjugates using cysteine-reactive PEGs and other cysteine-reactive moieties. The application teaches that certain amino acids in EPO are non-essential for biological activity and can be mutated to cysteine residues without altering the normal disulfide binding pattern and overall conformation of the molecule. Example substitutions contemplated under WO 99/03887 include S126C, N24C, I25C, T26C, N38C, I39C, T40C, N83C, S84C, A1C, P2C, P3C, R4C, D8C, S9C, T27C, G28C, A30C, E31C, H32C, S34C, N36C, D43C, T44C, K45C, N47C, A50C, K52C, E55C, G57C, Q58C, G77C, Q78C, A79C, Q86C, W88C, E89C, T107C, R110C, A111C, G113C, A114C, Q115C, K116C, E117C, A118C, S120C, P121C, P122C, D123C, A124C, A125C, A127C, A128C, T132C, K154C, T157C, G158C, E159C, A160C, T163C, G164C, D165C, R166C arid S85C.

The present invention discloses EPO analogues featuring substitutions at one or more of the above positions identified for free cysteine incorporation. However the present invention specifically teaches away from any of the contemplated substitutions presented above and is not at all focussed to the incorporation of free cysteine residues suitable for derivation with PEG moieties.

US patent 4,703,008 provides naturally occurring variants of EPO as well as amino acid substitutions that are present in EPO proteins of mammals.

EP0357804 provides EPO compositions comprising substitution of M54. The preferred substitution is M54L and another preferred embodiment specifies substitution at N38.
The M54L substitution is considered to render the EPO composition less susceptible to oxidation and minimises misincorporation of norleucine during biosynthesis.

Others still have provided modified EPO molecules, examples include US 5,856,298 and US 5,955,422, but it is understood that these approaches and other examples are directed towards improvements in the commercial production of EPO and approaches towards influencing the glycosylation status of the protein as a recombinant molecule.

None of these teachings recognise the importance of T cell epitopes to the immunogenic properties of the protein nor have been conceived to directly influence said properties in a specific and controlled way according to the scheme of the present invention.

The provenance or location of T-cell epitopes within a linear protein sequence is referred to herein as an "epitope map". It is an objective of the present invention to provide an epitope map for human EPO.

It is a further objective of the invention to provide EPO analogues in which the previously mapped T-cell epitopes are compromised in their ability to function as MHC class II ligands and or activate T-cells in combination with MHC class II molecules. It is highly desired to provide EPO with reduced or absent potential to induce an immune response in the human subject and it is therefore a particular objective of the present invention to provide modified EPO proteins in which the immune characteristic is modified by means of reduced numbers of potential T-cell epitopes, as claimed in claim 1.

In summary the invention relates to the following issues:
- an EPO molecule containing a modified peptide sequence which when individually tested evokes a stimulation index of less than 2.0 in a T-cell assay;
- an EPO molecule containing modifications such that when tested in a T-cell assay evokes a reduced stimulation index in comparision to a non modified protein molecule;
- an EPO molecule in which the immunogenic regions have been mapped using a T-cell assay and then modified such that upon re-testing in a T-cell assay the modified protein evokes a stimulation index smaller than the parental (non-modified) molecule and most preferably less than 2.0 or even less than 1.8.
- a modified molecule having the biological activity of EPO and being substantially non-immunogenic or less immunogenic than any non-modified molecule having the same biological activity when used *in vivo;*
- an accordingly specified molecule wherein alteration is conducted at one or more residues from the string of contiguous residues defined herein as epitope regions and comprising one of the sequences
   (a) RVLERYLLEAKEAENITTGCAEHCSLNENITVP,
   (b) RGQALLVNSSQPWEPLQLHVDKAVSGLRSLTTL, or
   (c) RTITADTFRKLFRVYSNFLRGKLKLYTGEACRT
- an accordingly specified molecule wherein alteration is conducted at one or more residues from the string of contiguous residues defined herein as epitope region R1 and comprising the sequence AKEAENITTGCAEHCSLNENI;
- an accordingly specified molecule wherein alteration is conducted at one or more residues from the string of contiguous residues defined herein as epitope region R2 and comprising the sequence RGQALLVNSSQPWEPLQLHVD;
- an accordingly specified molecule wherein alteration is conducted at one or more residues from the string of contiguous residues defined herein as epitope region R3 and comprising the sequence TFRKLFRVYSNFLRGKLKLYT;
- a DNA sequence or molecule which codes for any of said specified modified molecules as defined above and below;
- a pharmaceutical composition comprising a modified molecule having the biological activity of EPO;

### DETAILED DESCRIPTION OF THE INVENTION

The present invention provides examples of suitable substitutions within the most immunogenic regions of the parent molecule and such substitutions are considered embodiments of the invention.

Synthetic peptides are tested for their ability to evoke a proliferative response in human T-cells cultured *in vitro.* The T-cells are present within peripheral blood mononuclear cell (PBMC) layer readily obtainable by well known means from whole blood samples. Moreover the PBMC preparation contains physiological ratios ofT-cells and antigen presenting cells and is therefore a good source of materials with which to conduct a surrogate immune reaction *in vitro.* The inventors have established that in the operation of such an assay, a stimulation index closly approaching or exceeding 2.0 is a useful measure of induced proliferation. The stimulation index (SI) is conventionally derived by division of the proliferation score (e.g. counts per minute of radioactivity if using for example ³H-thymidine incorporation) measured to the test peptide by the score measured in cells not contacted with a test peptide. Peptides which evoke no response give SI =1.0 although in practice SI values in the range 0.8 - 1.2 are unremarkable. A number of technical proceedures can be inbuilt into the operation of such assays in order to ensure confidence in the recorded scores. Typically all determinations are made at least in triplicate and the mean score may be computed. Where a computed SI =>2.0 individual scores of the triplicate can be examined for evidence of outlying data. Test peptides are contacted with cells in at least two different concentrations and the concentrations would typically span a minimum two-fold concentration difference. Such a concentration range provides an off-set to the kinetic dimension to the assay and is especially important where a single time point determination, for example at plus day 7, is being conducted. In some assays multiple time course determinations may be conducted but in any event these too would be made using peptide immunogen provided at a minimum of two different concentrations.

Similarly the inclusion of control peptides for which there is expectation that the majority of PBMC donor samples will be responsive may be included in each assay plate. The influenza haemagglutinin peptide 307-309, sequence PKYVKQNTLKLA; and the *Chlamydia* HSP 60 peptide sequence KWDQIKKISKPVQH are particularly suitable control peptides although many other examples may be exploited. Assays should preferably also use a potent whole protein antigen such as hemocyanin from Keyhole Limpet to which all PBMC samples would be expected to exhibit an SI significantly greater than 2.0

It is particularly desired to provide an epitope map of human EPO where the map has relevance to a wide spectrum of possible MHC allotypes. It is desired that the map is sufficiently representative to allow the design or selection of a modified protein for which the ability of the protein to evoke a T-cell driven immune response is eliminated or at least ameliorated for the majority of patients to whom the protein is likely to be administered. Accordingly in the practice of the screening process, PBMC derived T-cells from naive donors is collected from a pool of donors of sufficient immunological diversity to provide a sample of at least greater than 90% of the MHC class II repertoire (HLA-DR) extant in the human population. Where a naive T-cell response is to be detected to a given synthetic peptide, the peptide in practice is contacted with PBMC preparations derived from multiple donors in isolation, the numbers of donors (or "donor pool" size), is for practical purposes not likely to be less than 20 unrelated individuals and all samples in the donor pool maybe pre-selected according to their MHC class II haplotype.
The term "naïve donor" in the context of the present invention means that the T-cells obtained from the individual who has not been in receipt of any therapeutic or exogenous sources of EPO.

The present invention herein discloses a method for T-cell epitope mapping exploiting immunologically naive T-cells. The T-cells are provided from a peripheral blood sample from a multiplicity of different healthy donors for whom the protein of interest may be an endogenous molecule but who have not been in receipt of the protein of interest from any exogenous source e.g. administered therapeutically. The assay is conducted using PBMC cultured *in vitro* using procedures common in the art and involves contacting the PBMC with synthetic peptide species representative of the protein of interest, and following a suitable period of incubation, measurement of peptide induced T cell activation such as cellular proliferation. Measurement is by any suitable means and may for example be conducted using ³H-thymidine incorporation whereby the accumulation of ³H into cellular material is readily measured using laboratory instruments. The degree of cellular proliferation for each combination of PBMC sample and synthetic peptide is examined relative to that seen in non peptide treated PBMC sample. Reference may also be made to the proliferative response seen following treatment with a peptide or peptides for which there is an expected proliferative effect. In this regard is considered particularly advantageous to use peptide with known broad MHC restriction and especially peptide epitopes with MHC restriction to the DP or DQ isotypes.

To facilitate assembly of an epitope map for human EPO, a set of synthetic peptides was produced. Each of the peptides was 15 amino acid residues in length and each overlapped the next peptide in the series by 12 amino acid residues; i.e. each successive peptide in the series incrementally added a further 3 amino acids to the analysis. In this way any given adjacent pair of peptides mapped 18 amino acids of contiguous sequence. For EPO a total of 51 peptides was required to enable a scan of the entire mature protein. A particularly effective method for defining a T-cell map for EPO using naive T-cell assays is provided in the EXAMPLE 1.

The present studies have uncovered 16 peptide sequences able to evoke a significant proliferative response. These peptides are listed in TABLE 1 and are an embodiment of the invention. Within this set of peptides, a further sub-set of peptides was identified, each peptide of which evoked a significant proliferative response in 2 or more individual donor samples. These peptides are listed in TABLE 2 and are a further embodiment of the invention.

**TABLE 1:**

| *EPO peptide sequences able to stimulate ex-vivo human T-cells.* | | |
|---|---|---|
| **Peptide ID#** | **Residue #*** | **Peptide Sequence** |
| P4 | 10 | RVLERYLLEAKEAEN |
| P7 | 19 | AKEAENITTGCAEHC |
| P8 | 22 | AENITTGCAEHCSLN |
| P9 | 25 | ITTGCAEHCSLNENI |
| P10 | 28 | GCAEHCSLNENITVP |
| P16 | 46 | VNFYAWKRMEVGQQA |
| P26 | 76 | RGQALLVNSSQPWEP |
| P27 | 79 | ALLVNSSQPWEPLQL |
| P28 | 82 | VNSSQPWEPLQLHVD |
| P32 | 94 | HVDKAVSGLRSLTTL |
| P41 | 122 | PDAASAAPLRTITAD |
| P44 | 131 | RTITADTFRKLFRVY |
| P46 | 137 | TFRKLFRVYSNFLRG |
| P47 | 140 | KLFRVYSNFLRGKLK |
| P48 | 143 | RVYSNFLRGKLKLYT |
| P50 | 149 | LRGKLKLYTGEACRT |

**TABLE 2:**

| *EPO peptide sequences able to stimulate ex vivo human T-cells from 2 or more donor samples* | | | |
|---|---|---|---|
| **Peptide ID #** | **Residue #** | **Peptide Sequence** | **Epitope Region** |
| P7 | 19 | AKEAENITTGCAEHC | R1 |
| P8 | 22 | AENITTGCAEHCSLN | |
| P9 | 25 | ITTGCAEHCSLNENI | |
| P26 | 76 | RGQALLVNSSQPWEP | R2 |
| P46 | 137 | TFRKLFRVYSNFLRG | R3 |
| P47 | 140 | KLFRVYSNFLRGKLK | |
| P50 | 149 | LRGKLKLYTGEACRT | |

Each of the peptides identified in TABLE 1 are suggested to be able to bind MHC class II and engage at least one cognate TCR with sufficient affinity to evoke a proliferative burst detectable in the assay system. For the peptides of TABLE 2 these exacting criteria have been achieved using PBMC derived from two or three unrelated PBMC samples. These peptides are considered to encompass the major epitope regions of the molecule and cluster to three zones in the EPO sequence termed herein epitope regions R1, R2 and R3.

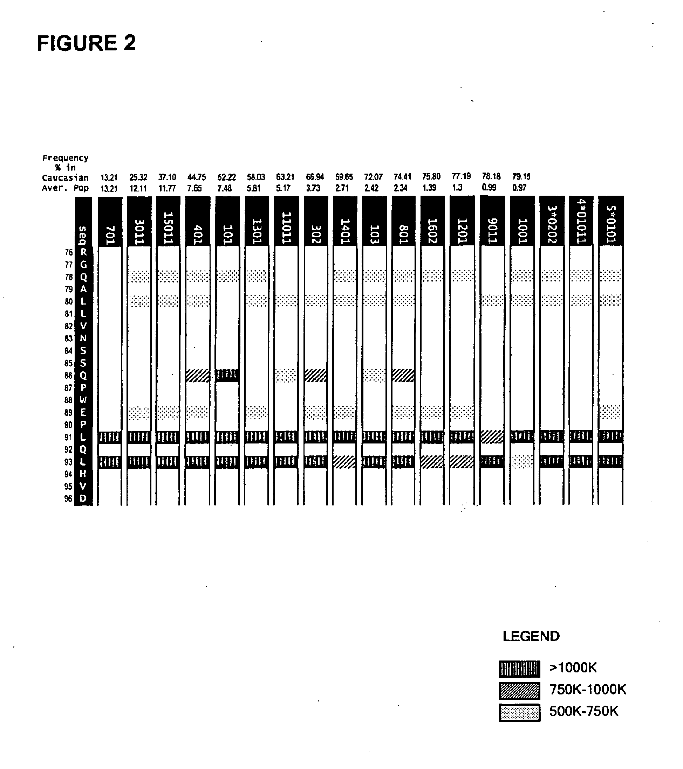

Epitope region R1 is encompassed by peptides P7, P8 and P9 comprising the sequence AKEAENITTGCAEHCSLNENI. Epitope region R2 is encompassed by peptide P26 comprising the sequence RGQALLVNSSQPWEP. Note that for the R2 epitope, successive peptides P27 and P28 are also reactive each with one PBMC donor sample. In the case of the P27 peptide the donor is also reactive to the P26 peptide and it is likely that a single common core sequence within R2 is responsible for this stimulation. Owing to the phasing of each successive peptide in the sequence, it is possible that the same core nonamer sequence is shared (i.e is common) between either 2 or 3 adjacent peptides. The exact phasing is dependent on proximity to the N-terminus and tied to the length of the peptides and number of "new" residues scanned by each successive increment of the sequence. In the case of the R2 epitope, the C-terminal boundary of the epitope region has been set to include sequence covered by peptides P27 and P28 not least as this region is shown to contain significant MHC class II ligands in its C-terminal region (see later and FIGURE 2). Epitope region R2 is accordingly defined by the sequence RGQALLVNSSQPWEPLQLHVD.

Epitope region R3 is encompassed by peptides P46 and P47 and extends toward the C-terminus of the EPO sequence. The C-terminal boundary of eptope R3 is limited by the natural terminus of the EPO protein, notably peptide P50 is also reactive in two donor samples and these donors are the same as react with peptides P46 and P47. The core of the R3 epitope is considered to comprise the sequence TFRKLFRVYSNFLRGKLK, but an additional MHC class II ligand and known reactive peptide comprises the overlapping P50 peptide sequence LRGKLKLYTGEACRT to give a total R3 sequence comprising TFRKLFRVYSNFLRGKLKLYT.

The disclosed peptide sequences herein represent the critical information required for the construction of modified EPO molecules in which one or more of these epitopes is compromised. Under the scheme of the present, the epitopes are compromised by mutation to result in sequences no longer able to function as T-cell epitopes. It is possible to use recombinant DNA methods to achieve directed mutagenesis of the target sequences and many such techniques are available and well known in the art.

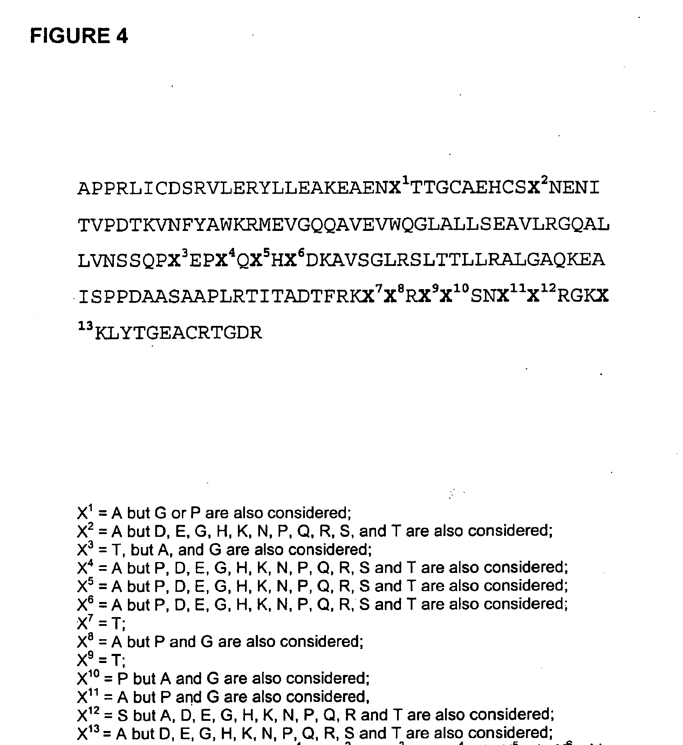

Where it is the objective of this invention to modify the amino acid sequences of at least one or more of the above listed peptides from TABLE 1, it is most preferred to modify the sequence of one or more of the peptides identified in TABLE 2. There are herein disclosed suitable modifications which achieve the objective of reducing or eliminating the capabilities of the subject peptide sequence to function as a T-cell epitope, at the level of being a ligand for one or more MHC class II allotypes. One such suitable set of modifications is provided in FIGURE 4.

According to this second embodiment, suitable modifications to the protein may include amino acid substitution of particular residues or combinations of residues. For the elimination of T-cell epitopes, amino acid substitutions are preferably made at appropriate points within the peptide sequence predicted to achieve substantial reduction or elimination of the activity of the T-cell epitope. In practice an appropriate point will preferably equate to an amino acid residue binding within one of the pockets provided within the MHC class II binding groove. It is most preferred to alter binding within the first pocket of the cleft at the so-called "P1" or "P1 anchor" position of the peptide. The quality of binding interaction between the P1 anchor residue of the peptide and the first pocket of the MHC class II binding groove is recognised as being a major determinant of overall binding affinity for the whole peptide. An appropriate substitution at this position of the peptide will be for a residue less readily accommodated within the pocket, for example, substitution to a more hydrophilic residue. Amino acid residues in the peptide at positions equating to binding within other pocket regions within the MHC binding cleft are also considered and fall under the scope of the present.

It is understood that single amino acid substitutions within a given potential T-cell epitope are the most preferred route by which the epitope may be eliminated. Combinations of substitution within a single epitope may be contemplated and for example can be particularly appropriate where individually defined epitopes are in overlap with each other. Moreover, amino acid substitutions either singly within a given epitope or in combination within a single epitope may be made at positions not equating to the "pocket residues" with respect to the MHC class II binding groove, but at any point within the peptide sequence. Substitutions may be made with reference to an homologous structure or structural method produced using *in silico* techniques known in the art and may be based on known structural features of the molecule. The EPO crystal structure model contained in the Protein Data Bank is particularly useful in this regard [PDB ID: 1CN4; Syed, R. et al (1998) Nature 395: 511-516]. A change may be contemplated to restore structure or biological activity of the variant molecule. Such compensatory changes and changes may also include deletion or addition of particular amino acid residues from the polypeptide.

A particularly effective means of removing epitopes from protein molecules is the concerted use of the naive T-cell activation assay scheme as outlined herein together with an *in silico* tool developed according to the scheme described in co-owned application WO 02/069232 which is also incorporated fully herein by reference.

The software simulates the process of antigen presentation at the level of the peptide MHC class II binding interaction to provide a binding score for any given peptide sequence. Such a score is determined for many of the predominant MHC class II allotypes extant in the population. As this scheme is able to test any peptide sequence, the consequences of amino acid substitutions additions or deletions with respect to the ability of a peptide to interact with a MHC class II binding groove can be predicted. Consequently new sequence compositions can be designed which contain reduced numbers of peptides able to interact with the MHC class II and thereby function as immmunogenic T-cell epitopes. Where the biological assay using any one given donor sample can assess binding to a maximum of 4 DR allotypes, the *in silico* process can test the same peptide sequence using >40 allotypes simultaneously. In practice this approach is able to direct the design of new sequence variants which are compromised in the their ability to interact with multiple MHC allotypes.

The T-cell assay was able to define three immunogenic regions R1- R3 within the molecule and the software system according to the scheme of WO 02/069232 was able to identify predicted MHC class II ligands within each of the epitopes. Moreover, the system was further able to identify amino acid substitutions within the epitopes which resulted in significant loss of binding affinity between the peptide sequence and essentially all of the MHC class II allotypes represented in the system.

One example of such a set of modifications is provided by the disruption of the R1 epitope region. The substitution set I25A and L35A result in compromise of the major MHC class II ligands within epitope R1.

Similarly for MHC class II ligands identified within epitope region R2, the substitutions V82A, Q88W, L91G, L93P and V95A are exemplary feasible changes.

For epitope region R3 an overlapping series of MHC ligands are identified. A suitable substitution series comprises one or more of the changes L141T, F142A, V144T, Y145P, F148A, L149S and or L153A. In all of the above instances, alternative mutation sets can be discerned based on the ability of a given peptide to bind within the MHC class II binding groove and structural considerations based on examination of the EPO crystal structure model [PDB ID: 1CN4; Syed, R. et al (1998) Nature 395: 511-516].

Each of the above substitutions are exemplary of the method and are preferred compositions under the scheme of the present invention. As will be clear to the person skilled in the art, multiple alternative sets of substitutions could be arrived at which achieve the objective of removing un-desired epitopes. The resulting sequences would however be recognised to be closely homologous with the specific compositions disclosed herein and therefore fall under the scope of the present invention. The mature protein form of EPO can contain 165 or 166 amino acids because of posttranslational removal of the C-terminal arginine. D165 is the C-terminus of the 165 amino acid form and R166 is the C-terminal amino acid of the 166 amino acid form. The EPO analogues described herein can comprise either the 165 or 166 amino acids forms of EPO.

The combined approach of using an *in silico* tool for the identification of MHC class II ligands and design of sequence analogues lacking MHC class II ligands, in concert with epitope mapping and re-testing optionally using biologically based assays of T-cell activation is a particularly effective method and most preferred embodiment of the invention. The general method according to this embodiment comprises the following steps:
i) use of naive T-cell activation assays and synthetic peptides collectively encompassing the protein sequence of interest to identify epitope regions capable of activating T-cells;
ii) use of a computational scheme simulating the binding of the peptide ligand with one or more MHC allotypes to analyse the epitope regions identified in step (i) and thereby identify MHC class II ligands within the epitope region;
iii) use of a computational scheme simulating the binding of the peptide ligand with one or more MHC allotypes to identify sequence analogues of the MHC ligands encompassed within the epitope region(s) which no longer bind MHC class II or bind with lowered affinity to a lesser number of MHC allotypes and optionally;
iv) use of naive T-cell activation assays and synthetic peptides encompassing entirely or in collection encompassing the epitope regions identified within the protein of interest and testing the sequence analogues in naïve T-cell activation assay in parallel with the wild-type (parental) sequences;

The term "T-cell epitope" means according to the understanding of this invention an amino acid sequence which is able to bind MHC class II, able to stimulate T-cells and / or also to bind (without necessarily measurably activating) T-cells in complex with MHC class II.

The term "peptide" as used herein and in the appended claims, is a compound that includes two or more amino acids. The amino acids are linked together by a peptide bond (defined herein below). There are 20 different naturally occurring amino acids involved in the biological production of peptides, and any number of them may be linked in any order to form a peptide chain or ring. The naturally occurring amino acids employed in the biological production of peptides all have the L-configuration. Synthetic peptides can be prepared employing conventional synthetic methods, utilizing L-amino acids, D-amino acids, or various combinations of amino acids of the two different configurations. Some peptides contain only a few amino acid units. Short peptides, e.g., having less than ten amino acid units, are sometimes referred to as "oligopeptides". Other peptides contain a large number of amino acid residues, e.g. up to 100 or more, and are referred to as "polypeptides". By convention, a "polypeptide" may be considered as any peptide chain containing three or more amino acids, whereas a "oligopeptide" is usually considered as a particular type of "short" polypeptide. Thus, as used herein, it is understood that any reference to a "polypeptide" also includes an oligopeptide. Further, any reference to a "peptide" includes polypeptides, oligopeptides, and proteins. Each different arrangement of amino acids forms different polypeptides or proteins. The number of polypeptides-and hence the number of different proteins-that can be formed is practically unlimited.

The EPO molecules of this invention can be prepared in any of several ways but is most preferably conducted exploiting routine recombinant methods. It is a relatively facile procedure to use the protein sequences and information provided herein to deduce a polynucleotide (DNA) encoding any of the preferred protein sequences. This can be achieved for example using computer software tools such as the DNSstar software suite [DNAstar Inc, Madison, WI, USA] or similar. Any such DNA sequence with the capability of encoding the preferred polypeptides of the present or significant homologues thereof, should be considered as embodiments of this invention.

As a general scheme, genes encoding any of the EPO protein sequences can be made using gene synthesis and cloned into a suitable expression vector. In turn the expression vector is introduced into a host cell and cells selected and cultured. The preferred molecules are purified from the culture medium and formulated into a preparation for therapeutic administration. Alternatively, a wild-type EPO gene sequence can be obtained for example following a cDNA cloning strategy using RNA prepared from suitable liver or kidney tissues or human cell lines. The wild-type gene can be used as a template for mutagenesis and construction of the preferred variant sequences. In this regard it is particularly convenient to use the strategy of "overlap extension PCR" as described by Higuchi et al [Higuchi et al (1988) Nucleic Acids Res. 16: 7351] although other methodologies and systems could be readily applied. The altered coding DNA is then expressed by conventional means in a selected host cell system from which the desired EPO is recovered and purified. Suitable host cells, purification and assay schemes are well known in the art and would include any of the schemes provided in WO 85/02610, WO 86/03520, WO 99/03887, EP0357804 or other examples.

Where constitution of the EPO molecule may be achieved by recombinant DNA techniques, this may include EPO molecules fused with other protein domains for example an antibody constant region domain. Methods for purifying and manipulating recombinant proteins including fusion proteins are well known in the art. Necessary techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987); "PCR: The Polymerase Chain Reaction", (Mullis et al., eds., 1994); "Current Protocols in Immunology" (J. E. Coligan et al., eds., 1991).

In as far as this invention relates to modified EPO, compositions containing such modified EPO proteins or fragments of modified EPO□ proteins and related compositions should be considered within the scope of the invention. A pertinent example in this respect could be development of peptide mediated tolerance induction strategies wherein one or more of the disclosed peptides is administered to a patient with immunotherapeutic intent. Accordingly, synthetic peptides molecules, for example one of more of those listed in TABLE 1 or more preferably sequences comprising all or part of any of the epitope regions R1 - R3 as defined above and featured in TABLE 2. Such peptides are considered embodiments of the invention.
In another aspect, the present invention relates to nucleic acids encoding modified EPO entities. In a further aspect the present invention relates to methods for therapeutic treatment of humans using the modified EPO proteins. In this aspect the modified EPO may be produced as a recombinant fusion protein.

The invention will now be illustrated by the experimental examples below. The examples refer to the following figures:

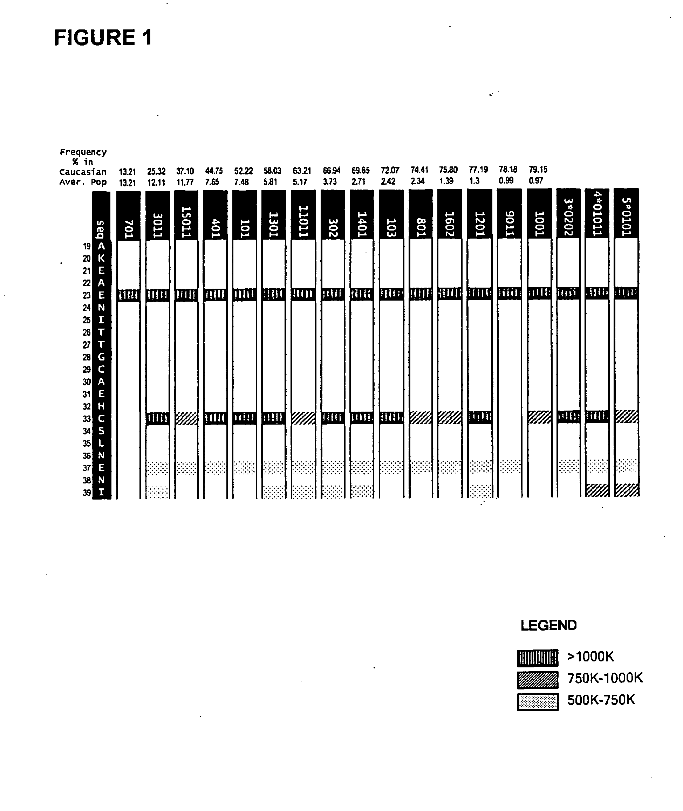

FIGURE 1 is a depiction of the MHC class II ligands identified within epitope region R1. Ligands are identified using the *in silico* system of EXAMPLE 2. In this case the binding profile of 18 human DR allotypes are displayed as columns. The ligands detected are 13-mers and residue number 1 of each 13-mer is identified by a coloured block. The intensity of the binding interaction (High, Medium or Low) for each peptide with respect to each of the 18 allotypes is indicated according to the key displayed.
FIGURE 2 is a depiction of the MHC class II ligands identified within epitope region R2. Ligands are identified using the *in silico* system of EXAMPLE 2. In this case the binding profile of 18 human DR allotypes are displayed as columns. The ligands detected are 13-mers and residue number 1 of each 13-mer is identified by a coloured block. The intensity of the binding interaction (High , Medium or Low) for each peptide with respect to each of the 18 allotypes is indicated according to the key displayed.

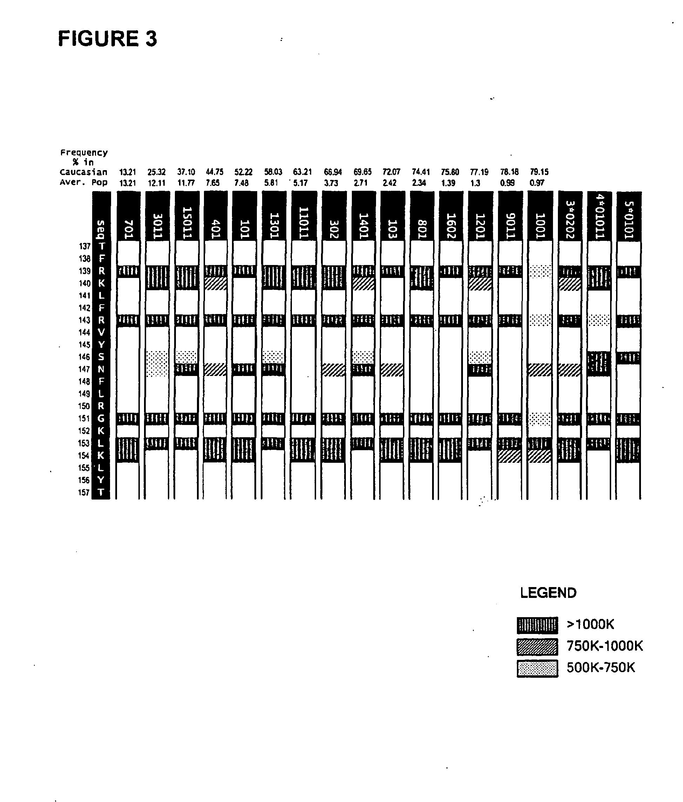

FIGURE 3 is a depiction of the MHC class II ligands identified within epitope region R3. Ligands are identified using the *in silico* system of EXAMPLE 2. In this case the binding profile of 18 human DR allotypes are displayed as columns. The ligands detected are 13-mers and residue number 1 of each 13-mer is identified by a coloured block. The intensity of the binding interaction (High , Medium or Low) for each peptide with respect to each of the 18 allotypes is indicated according to the key displayed.
FIGURE 4 depicts a most preferred EPO structure in which MHC class II ligands are eliminated by substitution within epitope regions R1, R2 and R3.

### EXAMPLE 1

The interaction between MHC, peptide and T-cell receptor (TCR) provides the structural basis for the antigen specificity of T-cell recognition. T-cell proliferation assays test the binding of peptides to MHC and the recognition ofMHC/peptide complexes by the TCR. *In vitro* T-cell proliferation assays of the present example, involve the stimulation of peripheral blood mononuclear cells (PBMCs), containing antigen presenting cells (APCs) and T-cells. Stimulation is conducted *in vitro* using synthetic peptide antigens, and in some experiments whole protein antigen. Stimulated T-cell proliferation is measured using ³H-thymidine (³H-Thy) and the presence of incorporated ³H-Thy assessed using scintillation counting of washed fixed cells.
Buffy coats from human blood stored for less than 12 hours were obtained from the National Blood Service (Addenbrooks Hospital, Cambridge, UK). Ficoll-paque was obtained from Amersham Pharmacia Biotech (Amersham, UK). Serum free AIM V media for the culture of primary human lymphocytes and containing L-glutamine, 50µg/ml streptomycin, 10µg/ml gentomycin and 0.1% human serum albumin was from Gibco-BRL (Paisley, UK). Synthetic peptides were obtained from Pepscan (The Netherlands) and Babraham Technix (Cambridge, UK).
Erythrocytes and leukocytes were separated from plasma and platelets by gentle centrifugation of buffy coats. The top phase (containing plasma and platelets) was removed and discarded. Erythrocytes and leukocytes were diluted 1:1 in phosphate buffered saline (PBS) before layering onto 15ml ficoll-paque (Amersham Pharmacia, Amersham UK). Centrifugation was done according to the manufacturers recommended conditions and PBMCs were harvested from the serum+PBS/ficoll paque interface. PBMCs were mixed with PBS (1:1) and collected by centrifugation. The supernatant was removed and discarded and the PBMC pellet resuspended in 50ml PBS. Cells were again pelleted by centrifugation and the PBS supernatant discarded. Cells were resuspended using 50ml AIM V media and at this point counted and viability assessed using trypan blue dye exclusion. Cells were again collected by centrifugation and the supernatant discarded. Cells were resuspended for cryogenic storage at a density of 3x10⁷ per ml.
The storage medium was 90%(v/v) heat inactivated AB human serum (Sigma, Poole, UK) and 10%(v/v) DMSO (Sigma, Poole, UK). Cells were transferred to a regulated freezing container (Sigma) and placed at -70°C overnight before transferring to liquid N₂ for long term storage. When required for use, cells were thawed rapidly in a water bath at 37°C before transferring to 10ml pre-warmed AIM V medium.
PBMC were stimulated with protein and peptide antigens in a 96 well flat bottom plate at a density of 2x10⁵ PBMC per well. PBMC were incubated for 7 days at 37°C before pulsing with ³H-Thy (Amersham-Phamacia, Amersham, UK). For the present study, synthetic peptides (15mers) that overlapped each successive peptide by 12 amino acids were generated to span the entire sequence of EPO. Peptide identification numbers (ID#) and sequences are given in TABLE 3.
Each peptide was screened individually against PBMC's isolated from 20 naïve donors. Two control peptides that have previously been shown to be immunogenic and a potent non-recall antigen KLH were used in each donor assay.
The control antigens used in this study were Flu haemagglutinin 307-319 (sequence: PKYVKQNTLKLAT); Chlamydia HSP 60 peptide (sequence: KVVDQIKKISKPVQH) and Keyhole Limpet hemocyanin.

**Table 3 EPO peptides**

| **Peptide ID#** | **EPO; 15mer peptide sequence** | **Residue #** |
|---|---|---|
| P1 | APPRLICDSRVLERY | 1 |
| P2 | RLICDSRVLERYLLE | 4 |
| P3 | CDSRVLERYLLEAKE | 7 |
| P4 | RVLERYLLEAKEAEN | 10 |
| P5 | ERYLLEAKEAENITT | 13 |
| P6 | LLEAKEAENITTGCA | 16 |
| P7 | AKEAENITTGCAEHC | 19 |
| P8 | AENITTGCAEHCSLN | 22 |
| P9 | ITTGCAEHCSLNENI | 25 |
| P10 | GCAEHCSLNENITVP | 28 |
| P11 | EHCSLNENITVPDTK | 31 |
| P12 | SLNENITVPDTKVNF | 34 |
| P13 | ENITVPDTKVNFYAW | 37 |
| P14 | TVPDTKVNFYAWKRM | 40 |
| P15 | DTKVNFYAWKRMEVG | 43 |
| P16 | VNFYAWKRMEVGQQA | 46 |
| P17 | YAWKRMEVGQQAVEV | 49 |
| P18 | KRMEVGQQAVEVWQG | 52 |
| P19 | EVGQQAVEVWQGLAL | 55 |
| P20 | QQAVEVWQGLALLSE | 58 |
| P21 | VEVWQGLALLSEAVL | 61 |
| P22 | WQGLALLSEAVLRGQ | 64 |
| P23 | LALLSEAVLRGQALL | 67 |
| P24 | LSEAVLRGQALLVNS | 70 |
| P25 | AVLRGQALLVNSSQP | 73 |
| P26 | RGQALLVNSSQPWEP | 76 |
| P27 | ALLVNSSQPWEPLQL | 79 |
| P28 | VNSSQPWEPLQLHVD | 82 |
| P29 | SQPWEPLQLHVDKAV | 85 |
| P30 | WEPLQLHVDKAVSGL | 88 |
| P31 | LQLHVDKAVSGLRSL | 91 |
| P32 | HVDKAVSGLRSLTTL | 94 |
| P33 | KAVSGLRSLTTLLRA | 97 |
| P34 | SGLRSLTTLLRALGA | 100 |
| P35 | RSLTTLLRALGAQKE | 103 |
| P36 | TTLLRALGAQKEAIS | 106 |
| P37 | LRALGAQKEAISPPD | 109 |
| P38 | LGAQKEAISPPDAAS | 112 |
| P39 | QKEAISPPDAASAAP | 115 |
| P40 | AISPPDAASAAPLRT | 118 |
| P41 | PDAASAAPLRTITAD | 122 |
| P42 | ASAAPLRTITADTFR | 125 |
| P43 | APLRTITADTFRKLF | 128 |
| P44 | RTITADTFRKLFRVY | 131 |
| P45 | TADTFRKLFRVYSNF | 134 |
| P46 | TFRKLFRVYSNFLRG | 137 |
| P47 | KLFRVYSNFLRGKLK | 140 |
| P48 | RVYSNFLRGKLKLYT | 143 |
| P49 | SNFLRGKLKLYTGEA | 146 |
| P50 | LRGKLKLYTGEACRT | 149 |
| P51 | KLKLYTGEACRTGDR | 152 |

Peptides were dissolved in DMSO to a final concentration of 10mM, these stock solutions were then diluted 1/500 in AIM V media (final concentration 20µM). Peptides were added to a flat bottom 96 well plate to give a final concentration of 2 and 20µM in a 100µl. The viability of thawed PBMC's was assessed by trypan blue dye exclusion, cells were then resuspended at a density of 2x10⁶ cells/ml, and 100µl (2x10⁵ PBMC/well) was transferred to each well containing peptides. Triplicate well cultures were assayed at each peptide concentration. Plates were incubated for 7 days in a humidified atmosphere of 5% CO₂ at 37°C. Cells were pulsed for 18-21 hours with 1µCi ³H-Thy/well before harvesting onto filter mats. CPM values were determined using a Wallac microplate beta top plate counter (Perkin Elmer). Results were expressed as stimulation indices, where the stimulation index (SI) is derived by division of the proliferation score (e.g. counts per minute of radioactivity) measured to the test peptide by the score measured in cells not contacted with a test peptide.

Mapping T cell epitopes in the EPO sequence using the T cell proliferation assay resulted in the identification of three immunogenic regions R1, R2 and R2. Peptides able to stimulate a significant response in at least one PBMC donor sample are listed within TABLE 1. Peptides able to stimulate a significant response in two or more PBMC donor samples are listed within TABLE 2. The allotypic restriction of responsive donors to EPO peptides is given in TABLE 4.

**TABLE 4**

| **Peptide ID** # | **Peptide Sequence** | **Responsive Allotypes** |
|---|---|---|
| P4 | RVLERYLLEAKEAEN | DRB 1*11, DRB 1*0103, DRB3 |
| P7 | AKEAENITTGCAEHC | DRB1*04, DRB1*07, DRB4*01 |
| | | DRBI*01, DRBI*08 |
| | | DRB1*10, DRB1*13, DRB3 |
| P8 | AENITTGCAEHCSLN | DPBI*01, DRB1*08 |
| | | DRB1*10, DRB1*13, DRB3 |
| | | DRB1*11, DRB1*15, DRB3, DRB5 |
| P9 | ITTGCAEHCSLNENI | DRB1*01, DRB1*08 |
| | | DRB1*10, DRB1*13, DRB3 |
| P10 | GCAEHCSLNENITVP | DRB1*01, DRB1*08 |
| P16 | VNFYAWKRMEVGQQA | DRB 1*11, DRB 1*0103, DRB3 |
| P26 | RGQALLVNSSQPWEP | DRB1*10, DRB1*13, DRB3 DRB1*11, DRB1*15, DRB3, DRB5 |
| P27 | ALLVNSSQPWEPLQL | DRB1*11, DRB1*15, DRB3, DRB5 |
| P28 | VNSSQPWEPLQLHVD | DRB1*10, DRB1*13, DRB3 |
| P32 | HVDKAVSGLRSLTTL | DRB1*04, DRB1*07, DRB4*01 |
| P41 | PDAASAAPLRT I TAD | DRB1*15, DRB1*0103, DRB5 |
| P44 | RTITADTFRKLFRVY | DRB1*11, DRB1*0103, DRB3 |
| P46 | TFRKLFRVYSNFLRG | DRB1*13, DRB1*14 or DRB1*14 only, DRB3 DRB1*11, DRB1*0103, DRB3 |
| P47 | KLFRVYSNFLRGKLK | DRB1*13, DRB1*14 or DRB1*14 only, DRB3 DRB1*11, DRB1*0103, DRB3 |
| P48 | RVYSNFLRGKLKLYT | DRB 1 * 11, DRB 1 *0103, DRB3 |
| P50 | LRGKLKLYTGEACRT | DRB1*13, DRB1*14 or DRB1*14 only, DRB3 DRB1*11, DRB1*0103, DRB3 |

### EXAMPLE 2

### Design of modified EPO sequences with improved immunogenicity profiles:

The method of co-owned application WO 02/069232 was used in an analysis of the epitope regions R1, R2 and R3. The system enables prediction of the particular MHC ligands encompassed within the biologically detected epitope regions and provides a "score" with respect to the ability of a given MHC class II ligand to interact with a particular MHC allotype.

The allotypic restriction pattern for the MHC ligands can be depicted using the allotypic restriction chart displays as provided for each of the epitope regions R1-R3 in the accompanying FIGURES 1-3.

The analysis was extended to consideration of sequence modifications within each of the epitopes R1-R3. The sequence variants were tested for continued ability bind MHC class II and their binding scores where these remained. Multiple amino acid substitutions were defined which achieved elimination of MHC class II binding with the majority of MHC allotypes tested. The particular substitutions identified were further tested for their ability to be accommodated within the structural model of the EPO molecule [PDB ID: 1CN4; Syed, R. et al (1998) Nature 395: 511-516]. Designed mutations on the selected residues of the wild type sequence were checked for steric clashes, hydrogen bonding formation, hydrophobic interactions and its general accommodation in the structure. Substitutions that gave rise to steric clashes were dismissed. Substitutions that were accommodated when the side chain was adopting a similar configuration (rotamer) to the original residue were considered acceptable. If more than one substitution fulfilled these criteria, residues that potentially form hydrogen bonds with neighboring side chains or backbone atoms, and/or form favourable hydrophobic contacts or other associations were preferred. The above procedure was performed interactively using Swiss Prot Deep View v3.7 [Guex, N. and Peitsch, M.C. (1997) Electrophoresis 18: 2714-2723]. This process resulted in a preferred substitution set for each of the epitope regions R1- R3. The substitution sets were compiled to produce the structure depicted in FIGURE 4. All substitutions were confirmed to result in removal of the MHC class II ligands within each of the epitope regions R1 - R3.

The EPO structure of the present invention contains the set of substitutions as depicted below and in FIGURE 4. 
APPRLICDSRVLERYLLEAKEAENX¹TTGCAEHCSX²NENITVPDTKVNFYAWKRMEVGQ
QAVEVWQGLALLSEAVLRGQALLVNSSQPX³EPX⁴QX⁵HX⁶DKAVSGLRSLTTLLRALGAQ
KEAISPPDAASAAPLRTITADTFRKX⁷X⁸RX⁹X¹⁰SNX¹¹X¹²RGKX¹³KLYTGEACRTGDR
 wherein
X¹ = A but G or P are also considered;
X² = A but D, E, G, H, K, N, P, Q, R, S, and T are also considered;
X³ = T, but A, and G are also considered;
X⁴ = A but P, D, E, G, H, K, N, P, Q, R, S and T are also considered;
X⁵ = A but P, D, E, G, H, K, N, P, Q, R, S and T are also considered;
X⁶ = A but P, D, E, G, H, K, N, P, Q, R, S and T are also considered;
X⁷ = T;
X⁸ = A but P and G are also considered;
X⁹=T;
X¹⁰ = P but A and G are also considered;
X¹¹ = A but P and G are also considered;
X¹² = S but A, D, E, G, H, K, N, P, Q, R and T are also considered;
X¹³ = A but D, E, G, H, K, N, P, Q, R, S and T are also considered;
As a preferred embodiment modified EPO molecule, wherein
X¹ = A, X² = A, X³ = T, X⁴ = A, X⁵ = A, X⁶ = A, X⁷ = T, X⁸ = A, X⁹ = T; X¹⁰ = P,
X¹¹ = A, X¹² = S, and X¹³ = A. is provided according to the invention.

## Claims

1. A modified molecule having the biological activity of human erythropoietin (EPO) and being substantially non-immunogenic or less immunogenic than any non-modified molecule having the same biological activity in an individual when used *in vivo,* wherein the said loss of immunogenicity is achieved by removing one or more T-cell epitopes derived from the originally non-modified molecule and said T-cell epitopes are MHC class II ligands or peptide sequences which show the ability to stimulate or bind T-cells via presentation on class II;
said modified EPO molecule has the amino acid sequence: 
APPRLICDSRVLERYLLEAKEAENX¹TTGCAEHCSX²NENITVPDTKVNFYAWKRMEVGQQAVEVW
QGLALLSEAVLRGQALLVNSSQPX³EPX⁴QX⁵HX⁶DKAVSGLRSLTTLLRALGAQKEAISPPDAAS
AAPLRTITADTFRKX⁷X⁸RX⁹X¹⁰SNX¹¹X¹²RGKX¹³KLYTGEACRTGDR
 wherein
X¹ = A, G, P;
X² = A, D, E, G, H, K, N, P, Q, R, S, and T;
X³ = T, A, and G;
X⁴ = A, P, D, E, G, H, K, N, P, Q, R, S and T;
X⁵ = A, P, D, E, G, H, K, N, P, Q, R, S and T;
X⁶ = A, P, D, E, G, H, K, N, P, Q, R, S and T;
X⁷ = T;
X⁸ = A, P and G;
X⁹ = T;
X¹⁰ = P, A and G;
X¹¹ = A, P and G;
X¹² = S, A, D, E, G, H, K, N, P, Q, R and T;
X¹³ = A, D, E, G, H, K, N, P, Q, R, S and T;
and,
when tested as a whole protein in a biological T-cell proliferation assay, exhibits a stimulation index (SI) smaller than 2.0 and smaller than the parental non-modified molecule.

2. A modified EPO molecule of claim 1, wherein
X¹=A, X²=A, X³=T,X⁴=A, X⁵ = A, X⁶ = A, X⁷ = T, X⁸ = A, X⁹ = T,X¹⁰ = P,
X¹¹= A, X¹² = S, and X¹³= A.

3. A DNA molecule coding for a modified EPO protein as specified in any of the claims 1 and 2.

4. A pharmaceutical composition comprising an EPO molecule as specified in any of the claims 1 and 2 together with a pharmaceutically acceptable carrier, diluent or excipient.

## Patentansprüche

1. Modifiziertes Molekül mit der biologischen Aktivität des humanen Erythropoetins (EPO), das im Wesentlichen nicht immunogen oder weniger immunogen als jegliches nicht-modifizierte Molekül mit der gleichen biologischen Aktivität in einem Individuum bei Verwendung *in vivo* ist, wobei dieser Verlust an Immunogenität durch das Entfernen von einem oder mehreren T-Zell-Epitopen erreicht wird, die von dem ursprünglichen, nicht-modifizierten Molekül abstammen, und wobei diese T-Zell-Epitope MHC-Klasse-II-Liganden oder Peptidsequenzen sind, die die Fähigkeit zeigen, T-Zellen über Präsentation auf Klasse II zu stimulieren oder zu binden;
wobei dieses modifizierte EPO-Molekül die folgende
Aminosäuresequenz aufweist: 
APPRLICDSRVLERYLLEAKEAENX1TTGCAEHCSX2NENITVPDTKVNFYAWKR
MEVGQQAVEVW
QGLALLSEAVLRGQALLVNSSQPX3EPX4QX5HX6DKAVSGLRSLTTLLRALGAQ
KEAISPPDAAS
AAPLRTITADTFRKX7X8RX9X10SNX11X12RGKX13KLYTGEACRTGDR
 wobei
X¹ = A, G, P;
X² = A, D, E, G, H, K, N, P, Q, R, S und T;
X³ = T, A und G;
X⁴ = A, P, D, E, G, H, K, N, P, Q, R, S und T;
X⁵ = A, P, D, E, G, H, K, N, P, Q, R, S und T;
X⁶ = A, P, D, E, G, H, K, N, P, Q, R, S und T;
X⁷ = T;
X⁸ = A, P und G;
X⁹ = T;
X¹⁰ = P, A und G;
X¹¹ = A, P und G;
X¹² = S, A, D, E, G, H, K, N, P, Q, R und T;
X¹³ = A, D, E, G, H, K, N, P, Q, R, S und T;
und,
wenn es als ein ganzes Protein in einem biologischen T-Zellproliferations-Assay untersucht wird, es einen Stimulationsindex (Sl) kleiner als 2,0 und kleiner als das parentale nicht-modifizierte Molekül aufweist

2. Modifiziertes EPO-Molekül nach Anspruch 1, in dem
X¹ = A, X² = A, X³ = T, X⁴ = A, X⁵ = A, X⁶ = A, X⁷ = T, X⁸ = A, X⁹ = T; X¹⁰ = P,
X¹¹ = A, X¹² = S und X¹³ = A

3. DNA-Molekül, kodierend für ein modifiziertes EPO-Protein wie nach einem der Ansprüche 1 und 2 spezifiziert.

4. Pharmazeutische Zusammensetzung, enthaltend ein EPO-Molekül, das in einem der Ansprüche 1 bis 2 spezifiziert wurde, zusammen mit einem pharmazeutisch unbedenklichen Träger, Verdünnungsmittel oder Hilfsstoff.

## Revendications

1. Molécule modifiée présentant l'activité biologique de l'érythropoïétine (EPO) humaine et étant sensiblement non immunogène ou moins immunogène qu'une quelconque molécule non modifiée présentant la même activité biologique chez un individu lorsqu'elle est utilisée *in vivo,* dans laquelle ladite perte d'immunogénicité est réalisée en enlevant un ou plusieurs épitopes de lymphocyte T dérivés à partir de la molécule originellement non modifiée et lesdits épitopes de lymphocyte T sont des ligands MHC de classe II ou des séquences de peptides qui présentent la capacité de stimuler ou de lier des lymphocytes T via une présentation sur la classe II;
ladite molécule d'EPO modifiée présente la séquence d'acides aminés: 
APPRLICDSRVLERYLLEAKEAENX1TTGCAEHCSX2NENITVPDTKVNFYAWKR
MEVGQQAVEVW
QGLALLSEAVLRGQALLVNSSQPX3EPX4QX5HX6DKAVSGLRSLTTLLRALGAQ
KEAISPPDAAS
AAPLRTITADTFRKX7X8RX9X10SNX11X12RGKX13KLYTGEACRTGDR
 dans laquelle
X¹ = A, G, P;
X² = A, D, E, G, H, K, N, P, Q, R, S et T;
X³ = T, A et G;
X⁴ = A, P, D, E, G, H, K, N, P, Q, R, S et T;
X⁵ = A, P, D, E, G, H, K, N, P, Q, R, S et T;
X⁶ = A, P, D, E, G, H, K, N, P, Q, R, S et T;
X⁷ = T;
X⁸ = A, P et G;
X⁹ = T;
X¹⁰ = P, A et G;
X¹¹ = A, P et G;
X¹² = S, A, D, E, G, H, K, N, P, Q, R et T;
X¹³ = A, D, E, G, H, K, N, P, Q, R, S et T;
et,
lorsqu'elle est testée en tant que protéine complète lors d'un test de prolifération de lymphocytes T biologique, elle présente un indice de stimulation (SI) inférieur à 2,0 et inférieur à celui de la molécule non modifiée parentale

2. Molécule d'EPO modifiée selon la revendication 1, dans laquelle
X¹ = A , X² = A , X³ = T , X⁴ = A , X⁵ = A , X⁶ = A , X⁷ = T , X⁸ = A , X⁹ = T; X¹⁰ = P,
X¹¹ = A, X¹² = S et X¹³ = A

3. Molécule d'ADN codant pour une protéine d'EPO modifiée selon l'une quelconque des revendications 1 et 2.

4. Composition pharmaceutique comprenant une molécule d'EPO telle que spécifiée selon l'une quelconque des revendications 1 et 2 en association avec un vecteur, un diluant ou un excipient acceptable pharmaceutiquement.
